(19) Europäisches Patentamt
European Patent Office
Office européen des brevets

(11) Veröffentlichungsnummer: **0 172 371**
**A1**

(12) # EUROPÄISCHE PATENTANMELDUNG

(21) Anmeldenummer: 85108212.3

(22) Anmeldetag: 03.07.85

(51) Int. Cl.⁴: C 07 D 315/00
C 07 C 121/34
//C07D327/10, C07C120/06

(30) Priorität: 27.07.84 CH 3660/84

(43) Veröffentlichungstag der Anmeldung: 26.02.86 Patentblatt 86/9

(84) Benannte Vertragsstaaten: AT BE CH DE FR GB IT LI NL

(71) Anmelder: F. HOFFMANN-LA ROCHE & CO. Aktiengesellschaft

CH-4002 Basel(CH)

(72) Erfinder: Fizet, Christian, Dr.
18 rue de Bruebach
F-68440 Zimmersheim(FR)

(74) Vertreter: Cottong, Norbert A. et al,
Grenzacherstrasse 124 Postfach 3255
CH-4002 Basel(CH)

(54) **Verfahren zur Herstellung von Dehydroxypantolacton.**

(57) Es wird ein neues Verfahren zur Herstellung des Furanons der Formel

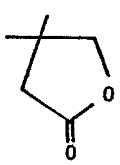

I

beschrieben. Hierbei wird zunächst ein Diol mit Thionylchlorid umgesetzt, dann das gebildete Sulfit, gegebenenfalls nach Oxidation zum entsprechenden Sulfat, mit Natriumcyanid behandelt und das entstehende Hydroxynitril hydrolysiert.

Das Furanon der Formel I kann als Ausgangsmaterial zur Herstellung von R-(-)-Pantolacton verwendet werden.

EP 0 172 371 A1

0172371

F.HOFFMANN-LA ROCHE & CO.Aktiengesellschaft, Basel/Schweiz

RAN 4224/58

# Verfahren zur Herstellung von Dehydroxypantolacton

Die vorliegende Erfindung betrifft ein neues Verfahren zur Herstellung des Furanons der Formel

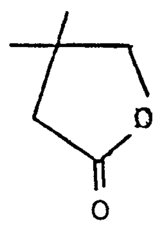

I

Dieses Furanon der Formel I ist eine bekannte Verbindung, welche unter anderem als Ausgangsmaterial für die Herstellung von optisch aktivem R-(-)-Pantolacton verwendet werden kann.

Das erfindungsgemässe Verfahren ist dadurch gekennzeichnet, dass man das Diol der Formel

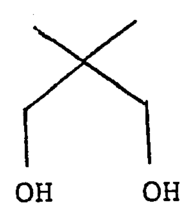

II

mit Thionylchlorid zur Verbindung der Formel

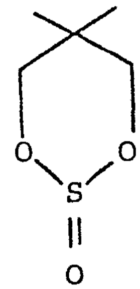

III

umsetzt, dass man diese gewünschtenfalls zur Verbindung der Formel

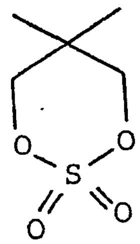

IV

oxidiert, dass man die Verbindung der Formel III oder IV mit Natriumcyanid umsetzt und dass man die so erhaltene Verbindung der Formel

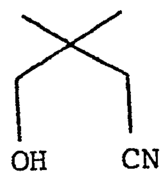

V

hydrolysiert.

Das als Ausgangsmaterial verwendete Diol der Formel II, sowie auch die Verbindungen der Formeln III und IV sind bekannte Verbindungen. Die Verbindung der Formel V ist jedoch neu und ebenfalls Gegenstand der vorliegenden Erfindung.

Die Umsetzung des Diols der Formel II mit Thionylchlorid zur Verbindung der Formel III kann in an sich bekannter Weise durchgeführt werden. Zweckmässig erfolgt diese Umsetzung in einem inerten organischen Lösungsmittel und bei einer Temperatur von etwa -20°C bis etwa Rückflusstemperatur des Reaktionsgemisches, vorzugsweise bei etwa 0°C bis etwa 60°C. Als Lösungsmittel können insbesondere genannt werden: halogenierte Kohlenwasserstoffe wie Methylenchlorid, Chloroform oder Tetrachlorkohlenstoff oder auch Aether wie Diäthyläther, Dioxan, Tetrahydrofuran und dergleichen.

Diese Umsetzung kann gewünschtenfalls auch in Abwesenheit eines Lösungsmittels durchgeführt werden.

Die Umsetzung der Verbindung der Formel III mit Natriumcyanid erfolgt in Dimethylsulfoxid und bei einer Temperatur von etwa 80°C bis etwa 120°C, vorzugsweise bei etwa 100°C. Die Umsetzung erfolgt auch zweckmässig unter Inertgas wie z.B. Argon, Stickstoff und dergleichen.

Die Hydrolyse der Verbindung der Formel V kann in an sich bekannter Weise, d.h. in zur Hydrolyse von Nitrilgruppen üblicher Weise erfolgen. Zweckmässig erfolgt dies durch Behandlung der Verbindung der Formel V mit einer Mineralsäure, beispielsweise Salzsäure, Schwefelsäure oder Phosphorsäure und dergleichen, bei Temperaturen bis etwa Rückflusstemperatur des Reaktionsgemisches. Die bei der Hydrolyse gebildete Carbonsäure lactonisiert unter den Reaktionsbedingungen spontan zum Furanon der Formel I.

Die Umsetzung der Verbindung der Formel III mit Natriumcyanid und die anschliessende Hydrolyse der so erhaltenen Verbindung der Formel V können sowohl wie vorhergehend beschrieben, als auch ohne Isolierung der Verbindung der Formel V erfolgen.

Die Oxidation der Verbindung der Formel III zur Verbindung der Formel IV kann in an sich bekannter Weise, d.h. in zur Oxidation von organischen Sulfiten analoger Weise erfolgen. Normalerweise erfolgt dies mittels Calciumpermanganat in wässrigem Medium. Ueberraschenderweise wurde nun auch gefunden, dass diese Oxidation im vorliegenden Fall auch mittels Kaliumpermanganat in Gegenwart von Phasentransferkatalysatoren durchgeführt werden kann. Als solche können hier beispielsweise genannt werden Benzyltriäthylammoniumchlorid oder auch Tetrabutylammoniumhydrogensulfat und dergleichen. Als organische Lösungsmittel können z.B. diejenigen verwendet werden, welche auch bei der Herstel-

0172371

lung der Verbindung III verwendbar sind. Weiterhin erfolgt die Oxidation auch zweckmässig bei einer Temperatur von etwa 0°C bis etwa 20°C, vorzugsweise bei etwa 5°-10°C.

Die Ueberführung der Verbindung der Formel IV in die Verbindung der Formel V und in diejenige der Formel I kann in zur Ueberführung der Verbindung der Formel III analoger Weise durchgeführt werden. Im vorliegenden Fall erfolgt die direkte Ueberführung, d.h. die Ueberführung ohne Isolierung der Verbindung der Formel V jedoch einfacher und es können ausser Dimethylsulfoxid auch noch andere polare Lösungsmittel wie etwa Aethylenglycol, Dimethylformamid, Diglyme und dergleichen verwendet werden.

Wie bereits erwähnt, ist das Furanon der Formel I eine bekannte Substanz und kann u.a. auch als Ausgangsmaterial für die Herstellung von optisch aktivem R-(-)-Pantolacton verwendet werden. Diese Ueberführung kann beispielsweise gemäss folgendem Reaktionsschema durchgeführt werden:

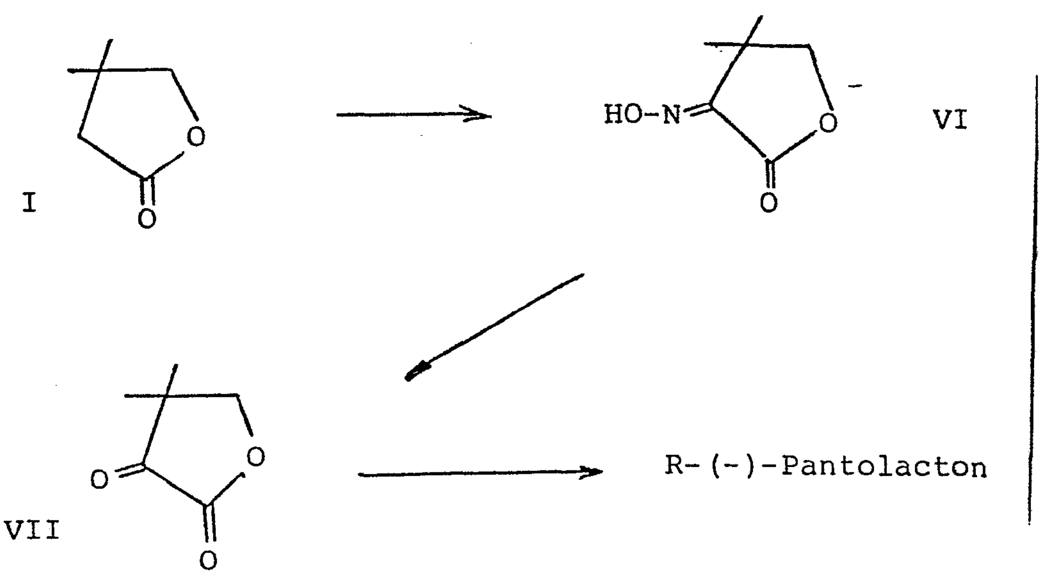


Die Oximbildung, d.h. die Ueberführung des Furanons der Formel I in das Oxim der Formel VI kann in an sich bekannter Weise, d.h. in zur Bildung von Oximen aus Estern analoger Weise erfolgen. Zweckmässig erfolgt dies durch Umsetzung mit einer starken Base wie einem Alkali- oder Erdalkalimetallhydrid, z.B. Lithiumhydrid, Natriumhydrid, Kaliumhydrid, Calciumhydrid und dergleichen.Ferner kommen auch Alkalimetallamide wie etwa Natriumamid, Kaliumamid und dergleichen in Frage. Die Menge an Base beträgt zweckmässig von etwa 0,9 bis etwa 2 und vorzugsweise etwa 1,4 Aequivalente. Die Oximbildung erfolgt auch zweckmässig in einem inerten organischen Lösungsmittel und bei einer Temperatur von etwa 0°C bis etwa 40°C, vorzugsweise von etwa 5°C bis etwa 10°C. Als Lösungsmittel können hier beispielsweise genannt werden: aliphatische oder aromatische Kohlenwasserstoffe wie etwa Hexan, Benzol, Toluol und dergleichen oder auch Aether wie etwa Diäthyläther, Dioxan und dergleichen. Ein bevorzugtes Lösungsmittel ist Hexan. Weiterhin erfolgt die Oximbildung auch in Gegenwart der üblichen Nitrite, vorzugsweise in Gegenwart von niederen Alkylnitriten mit etwa 1 bis 5 Kohlenstoffatomen. Die Menge an Nitrit beträgt zweckmässig von etwa 1 bis etwa 2 und vorzugsweise etwa 1,1 bis 1,2 Aequivalente.

Die Ueberführung des Oxims der Formel VI in das Ketopantolacton der Formel VII kann in an sich bekannter Weise durchgeführt werden. Ueberraschenderweise hat sich jedoch herausgestellt, dass im vorliegenden Fall diese Ueberführung auch durch einfache Zugabe einer Mineralsäure erfolgt, wie etwa Salzsäure oder Schwefelsäure und bei einer Temperatur von etwa 0°C bis etwa 40°C, vor zugsweise bei etwa Raumtemperatur. Es hat sich nämlich gezeigt, dass nach der Zugabe der Mineralsäure zum Oxim, das sich bildende Ketopantolacton ohne weiteres mit einem geeigneten organischen Lösungsmittel extrahiert werden kann. Als besonders geeignete Lösungsmittel können beispielsweise genannt werden: halogenierte Kohlenwasserstoffe wie Methylenchlorid oder Tetrachlorkohlenstoff oder auch aromatische Kohlenwasserstoffe wie etwa Benzol, Toluol, Xylol

und dergleichen. Vorzugsweise werden Methylenchlorid und Toluol verwendet.

Das Oxim der Formel VI kann nach seiner Bildung aus dem Reaktionsgemisch isoliert werden oder auch in situ weiter verarbeitet werden.

Das Ketopantolacton der Formel VII ist eine bekannte Verbindung und kann in bekannter Weise, z.B. durch asymmetrische Hydrierung, in R-(-)-Pantolacton übergeführt werden.

## Beispiel 1

In einem 500 ml Vierhalskolben, versehen mit Rührer, Rückflusskühler, Thermometer und Tropftrichter, werden 104,15 g 2,2-Dimethyl-1,3-Propandiol (1 Mol) in 200 ml Methylenchlorid suspendiert. Unter Rühren werden bei 5°-10°C 125 g (1,05 Mol) Thionylchlorid innert 1 Stunde zugetropft. Dann wird das Reaktionsgemisch 3 Stunden auf 40°C erwärmt. Das so erhaltene Oel wird mit 50 ml Wasser, dann mit 50 ml $NaHCO_3$-Lösung gewaschen und über $Na_2SO_4$ getrocknet. Nach Entfernen der Hauptmenge an Methylenchlorid am Rotationsverdampfer wird der Rückstand bei 85°-87°C/30 mmHg über eine 10 cm, mit Raschig-Ringen gefüllte, isolierte Kolonne destilliert. Man erhält 142,5 g 5,5-Dimethyl-1,3,2-dioxathian 2-oxid in Form eines farblosen Oeles.

## Beispiel 2

In einen 350 ml Vierhalskolben, versehen mit Rührer, Rückflusskühler und Thermometer, werden unter Argon-Begasung 30 g (0,198 Mol) 5,5-Dimethyl-1,3,2-dioxathian 2-oxid (hergestellt gemäss Beispiel 1) und 15,5 g (0,317 Mol) trockenes, fein pulverisiertes NaCN in 40 ml trockenem Dimethylsulfoxid gegeben. Das Reaktionsgemisch wird 30-36 Stunden bei 100°C gerührt. Dann wird die Hauptmenge des Dimethylsulfoxids (35-36 ml) bei 65°-70°C/3 mmHg über eine

5 cm Vigreux-Kolonne vorsichtig abdestilliert. Der Rückstand wird in 130 ml Wasser aufgeschlämmt und mit ca. 16 ml 25%iger HCl-Lösung auf pH 3 gebracht. Das schwarze Gemisch wird mit 80 ml Isopropyläther kontinuierlich extrahiert. Nach dem Trocknen über $Na_2SO_4$ und Entfernung des Lösungsmittels am Rotationsverdampfer wird das Produkt über eine 5 cm Vigreux-Kolonne bei 75°-82°C/0,2 mmHg destilliert. Man erhält 16 g 4-Hydroxy-3,3-dimethyl-butyronitril in Form eines Oeles.

## Beispiel 3

In einem 750 ml Vierhalskolben, versehen mit Rührer, Rückflusskühler und Thermometer, werden 79,4 g (0,701 Mol) 4-Hydroxy-3,3-dimethyl-butyronitril (hergestellt gemäss Beispiel 2) und 400 ml 25%ige HCl-Lösung vorgelegt. Das Gemisch wird 5 Stunden bei 75°C gerührt und dann kontinuierlich mit 200 ml Methylenchlorid extrahiert. Nach Trocknung über $Na_2SO_4$ wird das Lösungsmittel am Rotationsverdampfer eingeengt. Die so erhaltene Rohware wird über eine Hickmann-Apparatur bei 71°-76°C/8 mmHg destilliert. Man erhält 76 g 4,5-Dihydro-4,4-dimethyl-2(3H)-furanon als glasige Substanz.

## Beispiel 4

In einem 350 ml Vierhalskolben, versehen mit Rührer und Thermometer, werden 10 g (0,067 Mol) 5,5-Dimethyl-1,3,2--dioxathian 2-oxid (hergestellt gemäss Beispiel 1), 70 ml Methylenchlorid, 0,76 g (0,0033 Mol) Benzyltriäthylammoniumchlorid und 70 ml Wasser vorgelegt. Bei 5°-10°C und unter starkem Rühren werden portionsweise 7,5 g (0,0475 Mol) $KMnO_4$ zugegeben. Das Reaktionsgemisch wird am Ende noch 30 Minuten bei Raumtemperatur gerührt und abgenutscht. Die organische Phase wird abgetrennt. Der Rückstand wird in der Nutsche mit 20 ml Methylenchlorid gut gewaschen und die wässrige Phase mit diesem neuen Filtrat nochmals extrahiert. Die vereinigten organischen Phasen werden mit 1 g $NaHSO_3$ in 20 ml Wasser ausgeschüttelt und mit wenig Wasser gewaschen. Nach dem Trocknen über $Na_2SO_4$ und Eindampfen erhält man 10,2 g 5,5-Dimethyl-1,3,2-dioxathian 2,2-dioxid als weisses Pulver mit einem Smp. von 80°-82°C.

## Beispiel 5

In einem 750 ml Vierhalskolben, versehen mit Rührer, Kühler und Thermometer, werden 45 g (0,271 Mol) 5,5-Dimethyl-1,3,2-dioxathian 2,2-dioxid und 23,9 g (0,49 Mol) trockenes, fein pulverisiertes NaCN in 200 ml Aethylenglycol vorgelegt. Dieses Gemisch wird 15 Stunden bei 75°C gerührt. Dann werden bei Raumtemperatur 400 ml 36%ige HCl-Lösung zugefügt. Anschliessend erwärmt man 4 Stunden bei 75°C. Das Reaktionsgemisch wird auf Raumtemperatur abgekühlt und ab- genutscht. Das Filtrat wird viermal mit je 150 ml Methylenchlorid extrahiert. Die vereinigten organischen Phasen werden zweimal mit je 50 ml Wasser gewaschen, über $Na_2SO_4$ getrocknet und eingedampft. Die so erhaltene Rohware wird bei 87°-92°C/18 mmHg über eine 4 cm Vigreux-Kolonne destilliert. Man erhält 26,1 g 4,5-Dihydro-4,4-dimethyl-2(3H)-furanon als glasige Substanz.

## Beispiel 6

In einen 250 ml Vierhalskolben, versehen mit Rührer, Thermometer, 100 ml Tropftrichter und Trockeneiskühler, werden unter Argonbegasung 15 g (131,4 mMol) 4,5-Dihydro--4,4-dimethyl-2(3H)-furanon (hergestellt gemäss Beispiel 3 oder 5) und 5,88 g (184 mMol, 1,4 Aeq.) Natriumhydrid 75% in 140 ml Hexan aufgeschlämmt. Bei 10°-15°C tropft man innert 15 Minuten 10,84 g (145 mMol, 1,1 Aeq.) Aethylnitrit in 70 ml Hexan gelöst dazu. Dann lässt man das Reaktionsgemisch sich langsam erwärmen, wobei eine regelmässige Entwicklung von ca. 3 Liter Wasserstoff innert 2-4 Stunden entsteht. Wenn nötig, wird die Temperatur mittels Eis bei

30°C gehalten. Das Reaktionsgemisch wird zu 150 ml 5%ige HCl rasch zugetropft und 15 Minuten gerührt. Die Hexanphase wird abgetrennt und die wässrige Phase viermal mit 100 ml Methylenchlorid extrahiert. Die vereinigten organischen Phasen werden über $Na_2SO_4$ getrocknet und am Rotationsverdampfer eingedampft. Man erhält 17,1 g Rohware die 14,2 g (E)-Dihydro-4,4-dimethyl-2(3H)-furanon-3-oxim und 2,6 g Edukt enthält. Dieses Edukt wird mittels Kugelrohrdestillation bei 70°-80°C/0,1 mmHg entfernt. Der kristalline Rückstand wird mit ca. 60 ml Toluol aufgeschlämmt, abgesaugt und getrocknet. Man erhält 13,1 g reines Produkt mit einem Schmelzpunkt von 170°-173°C.

## Beispiel 7

In einen 500 ml Rundkolben mit Magnetrührer, werden 25 g (E)-Dihydro-4,4-dimethyl-2(3H)-furanon-3-oxim (hergestellt gemäss Beispiel 6) in 250 ml 37%ige HCl gegeben. Das Gemisch wird während 2 Stunden gerührt und dann mittels eines Keberle-Extraktors mit Methylenchlorid extrahiert. Nach Trocknung über $Na_2SO_4$ wird das Lösungsmittel am Rotationsverdampfer entfernt, wobei ein Rohprodukt kristallisiert. Dieses Rohprodukt wird bei 63°-66°C/0,1 mmHg in einer mit Luftkühler versehenen Apparatur destilliert und man erhält 18,9 g Dihydro-4,4-dimethyl-2,3-furandion mit einem Schmelzpunkt von 64°-67°C.

## Patentansprüche

1. Verfahren zur Herstellung des Furanons der Formel

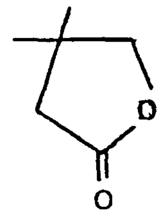

I

dadurch gekennzeichnet, dass man das Diol der Formel

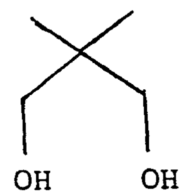

II

mit Thionylchlorid zur Verbindung der Formel

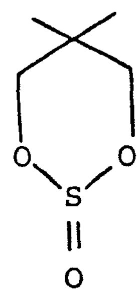

III

umsetzt, dass man diese gewünschtenfalls zur Verbindung der Formel

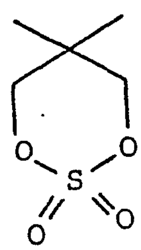

IV

oxidiert, dass man die Verbindung der Formel III oder IV mit Natriumcyanid umsetzt und dass man die so erhaltene Verbindung der Formel

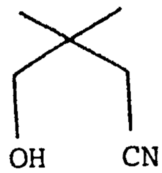

V

hydrolysiert.

2. Verfahren gemäss Anspruch 1, dadurch gekennzeichnet, dass man die Umsetzung der Verbindung der Formel III mit Natriumcyanid in Dimethylsulfoxid durchführt.

3. Verfahren gemäss Anspruch 2, dadurch gekennzeichnet, dass man die Umsetzung bei einer Temperatur von etwa 80°C bis etwa 120°C, vorzugsweise bei etwa 100°C durchführt.

4. Die Verbindung der Formel

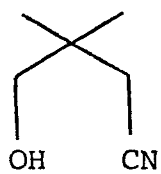

V

***

## Patentansprüche für Oesterreich

1. Verfahren zur Herstellung des Furanons der Formel

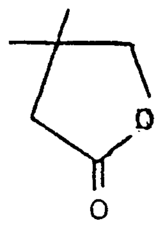

I

dadurch gekennzeichnet, dass man das Diol der Formel

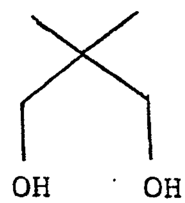

II

mit Thionylchlorid zur Verbindung der Formel

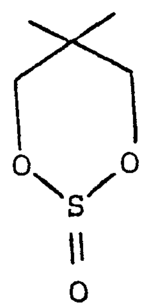

III

umsetzt, dass man diese gewünschtenfalls zur Verbindung der Formel

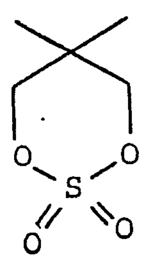

IV

oxidiert, dass man die Verbindung der Formel III oder IV mit Natriumcyanid umsetzt und dass man die so erhaltene Verbindung der Formel

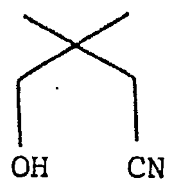

V

hydrolysiert.

2. Verfahren gemäss Anspruch 1, dadurch gekennzeichnet, dass man die Umsetzung der Verbindung der Formel III mit Natriumcyanid in Dimethylsulfoxid durchführt.

3. Verfahren gemäss Anspruch 2, dadurch gekennzeichnet, dass man die Umsetzung bei einer Temperatur von etwa 80°C bis etwa 120°C, vorzugsweise bei etwa 100°C durchführt.

***

Europäisches Patentamt

**EUROPÄISCHER RECHERCHENBERICHT**

0172371
Nummer der Anmeldung
EP 85 10 8212

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| A | HOUBEN-WEYL, METHODEN DER ORGANISCHEN CHEMIE, (1963), Band VI/2, Seiten 656-657; "Synthese und Cyclisierung von Hydroxy-carbonsäurenitrilen"<br>--- | 1-3 | C 07 D 315/00<br>C 07 C 121/34 //<br>C 07 D 327/10<br>C 07 C 120/06 |
| A | BE-A- 718 243 (ASPRO-NICHOLAS LIMITED)<br>* Seite 2, erster Absatz *<br>----- | 1 | |
| | | | RECHERCHIERTE SACHGEBIETE (Int Cl 4) |
| | | | C 07 D 315/00<br>C 07 C 121/00<br>C 07 C 120/00 |

Der vorliegende Recherchenbericht wurde für alle Patentansprüche erstellt.

| Recherchenort | Abschlußdatum der Recherche | Prüfer |
|---|---|---|
| DEN HAAG | 04-10-1985 | VERHULST W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82